# EUROPEAN PATENT APPLICATION

(11) **EP 4 026 658 A1**
(43) Date of publication of application: **13.07.2022**
(21) Application number: 21000367.9
(22) Date of filing: 28.12.2021
(51) Int. Cl.: B25J 5/00, A47B 31/00, A61B 50/13, B25J 11/00, B62B 3/00, G05D 1/02

(54) **ROBOTIC PLATFORM IN THE FORM OF A MULTIFUNCTIONAL ANCILLARY TABLE**

(30) Priority: 31.12.2020 PL 43660520
(71) Applicant: Politechnika Slaska, 44-100 Gliwice (PL)
(72) Inventor: Chuchnowska, Iwona, 44-100 Gliwice (PL); Bialas, Katarzyna, 44-100 Gliwice (PL); Labus, Agnieszka, 41-943 Piekary Slaskie (PL); Benek, Iwona, 43-400 Cieszyn (PL); Dapa, Patrycja, 44-100 Gliwice (PL); Dziadula, Wiktoria, 43-600 Jaworzno (PL); Franitza, Pawel, 41-800 Zabrze (PL); Stlarczyk, Michal, 40-748 Katowice (PL); Niedzwiedz, Sandra, 47-370 Kujawy (PL); Sladczyk, Sandra, 44-213 Ksiazenice (PL)

(57) **Abstract**

Robotic platform is made in the form of a multifunctional table equipped with a self-propelled base, which consists of a vertical frame supporting the utility part which contains drawers, cabinets, lockers, niches and a worktop characterized that the utility part has essential characteristic features, such as: a lower level (2) situated directly on the self-propelled base (1) equipped with a camera (6), a middle level (3) located above the lower level (2), an upper level (4) located between the middle level (3) and a multipart worktop (5). The platform (A) is wirelessly integrated with the control unit (B). The lower level (2) is equipped with drawers (2a).

## Description

The subject of the invention is a robotic platform in the form of a multifunctional ancillary table, especially for dependable and elderly people. The device facilitates daily activities and social communication.

The ancillary table may be used by individual persons who are able to function independently or by the dependent ones in places such as hospitals, care homes, etc. The device may be used by an independent person, nursing staff or carers of a dependent person.

There already exist devices typically used in hospitals or care homes playing a function of the control centre of an elderly person, however, this multifunctional ancillary table meets every user's needs and serves its purpose in various places, such as: hospitals, care homes or private homes.

Taking into account the current state of technology, there is a utility pattern of the following application number W.1 13658 and application P. 341223.

The essence of the invention is a robotic platform in the form of a multifunctional table equipped with a self-propelled base which consists of a vertical frame supporting the utility part which contains drawers, cabinets, lockers, niches and a worktop, characterized that the utility part has essential characteristic features, such as: a lower level - situated directly on the self-propelled base, equipped with a camera, a middle level - located above the lower level, an upper level - located between the middle level and a multipart worktop. In addition, the platform is wirelessly integrated with the control unit.

The lower level is equipped with drawers. The middle level is equipped with drawers and niches. The upper level is equipped with drawers and niches.

The multipart worktop is expandable and has a niche / opening for a smartphone. The control unit is a computer or a wireless phone.

Robotic platform is provided with side supports.

Robotic platform has a cylindrical shape.

The purpose of designing a modern robot in the form of a traditional coffee table of a cylindrical shape is to blend it into the interior where an elderly person is staying. The robotic platform is able to move around the room by presetting a position to be reached or by a manual remote control. The multifunctional table may be helpful in preventive treatment (prophylaxis) and health monitoring by displaying messages or programming certain functions. The robot will remind the patient about blood pressure measurements and record the results, or remind the patient about drinking a proper amount of water. The table can also move to a certain spot, enable video conferences, provide space for medicines storage and daily-use equipment. It also makes it possible to expand the worktop to obtain more working surface. The platform can serve as a self-propelled, stabile table, at the same time playing an organizational function for everyday activities.

Apart from that, it can finally serve the purpose of interaction with the patient, as a diary of physical activity, database of information on the patient's health, including blood pressure readouts or the history of the user's wellbeing. The main screen of the application enables the selection of activities and links the user to further sub-functions.

Robotic platform in the form of a multifunctional ancillary table may be helpful in care homes or hospitals. The carers of dependent persons may control the table in order to improve and facilitate their work. The table may move to certain patients simultaneously transporting water or medicines.

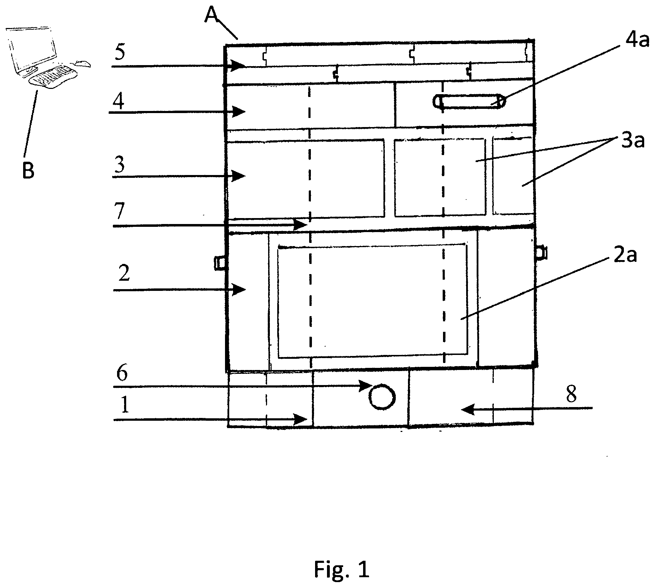

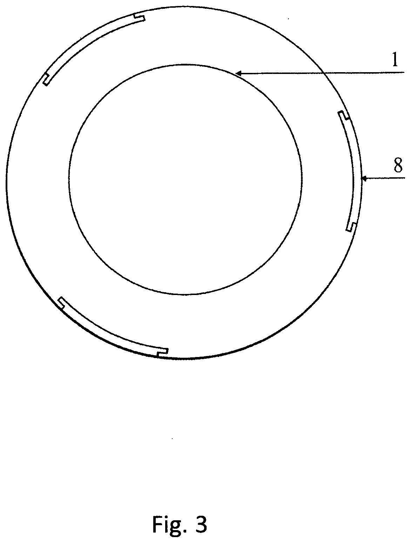

In the case of its use by independent persons, it can be useful for transport of small utility objects. The designed application may be helpful in reminding the patient about coming events, administration of medicines and medical check-ups. The invention has been presented in illustrations, where Figure 1 shows a side view of the platform, Figure 2 - the worktop folded and expanded, Figure 3-frog's eye view of the invention, Figure 4 - presents the visualization of the robotic platform.

The examples of the invention applications have been presented.

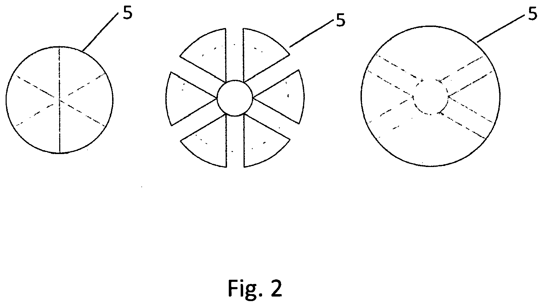

Robotic platform (A) is made in the in the form of a multifunctional table consisting of a self-propelled base (1) equipped with a camera (6). Above the self-propelled base (1) there is a lower level (2), which is the highest in relation to other levels for the purpose of keeping the centre of gravity in place. This level is equipped with closed drawers (2a). Above them there is a middle level (3), which features drawers and niches (3a). Between the middle level (3) and a worktop (5) there is an upper level (4) including drawers and niches (4a) providing space for the user's items. The worktop (5) is multipart and expandable. In a folded version (Fig. 2), its diameter is the same as the diameter of lower levels. The parts of the worktop (5) slide to expand, which as a result increases its usable surface. The worktop (5) is divided into six equal parts which can be slid on slideways and the worktop can be expanded. The empty space is then supplemented with other elements which complete the whole worktop. The whole concept is shown in Fig. 2. The worktop (5) features an opening for a smartphone.

Above the self-propelled base (1) there is a core which holds particular levels of the table including the worktop (5). The frame (7) of the platform (A) is made from flat steel bars, which constitute the internal structure of the platform.

Side supports (8) serve the purpose of stabilization of the table on slideways. The operation of the platform is started by the control unit, in this case a computer (B), where the software programme necessary for the operation was installed. The software programme controls the operation and mobility of the platform using an application for tele-operation and wireless communication with the robotic platform based on the framework of ROS melodic / ROS kinetic.

The camera (6) rotates to examine the way the platform should take and detects possible obstacles. The camera (6) operates as a vision sensor and transmits information about such an obstacle to the control unit (B), which causes the robotic platform to stop and prevents thus a collision.

The application enables, among other things, the monitoring of the camera view, current mapping of the environment, manual control through the input of a virtual controller as well as an automatic planning of the route by means of clicking on the interactive map displaying the location of the robot in a real-life time.

In addition to that, it can finally serve the purpose of interaction with the patient, as the diary of the patient's daily physical activity, database of the patient's health information, including the patient's blood pressure readouts or well-being history.

## Claims

1. Robotic platform is made in the form of a multifunctional table equipped with a self-propelled base, consists of a vertical frame supporting the utility part which contains drawers, cabinets, lockers, niches and a worktop **characterized that** the utility part has essential characteristic features, such as: a lower level (2) situated directly on the self-propelled base (1) equipped with a camera (6), a middle level (3) located above the lower level (2), an upper level (4) located between the middle level (3) and a multipart worktop (5), in addition, the platform (A) is wirelessly integrated with the control unit (B).

2. Robotic platform, acc. to claim 1, **characterized that** lower level (2) is equipped with drawers (2a).

3. Robotic platform, acc. to claim 1, **characterized that** a middle level (3) is equipped with drawers and niches (3a).

4. Robotic platform, acc. to claim 1, **characterized that** an upper level (4) is equipped with drawers and niches (4a).

5. Robotic platform, acc. to claim 1, **characterized that** a multipart worktop (5) is expandable.

6. Robotic platform, acc. to claim 1, **characterized that** in the worktop (5) there is a niche / opening for a smartphone.

7. Robotic platform, acc. to claim 1, **characterized that** the control unit (B) is controlled wirelessly by a computer or a smartphone.

8. Robotic platform, acc. to claim 1, **characterized that** there are side supports (8).

9. Robotic platform, acc. to claim 1, **characterized that** its essential characteristic shape is cylindrical.
